(19) Europäisches Patentamt
European Patent Office
Office européen des brevets

(11) Veröffentlichungsnummer: **0 101 933**
**A1**

(12) # EUROPÄISCHE PATENTANMELDUNG

(21) Anmeldenummer: **83107354.9**

(22) Anmeldetag: **27.07.83**

(51) Int. Cl.[3]: **C 07 D 279/16**
C 07 C 149/42, C 07 C 149/43
A 61 K 31/54
//A23B4/14, A23K1/00

(30) Priorität: **04.08.82 DE 3229124**
**04.08.82 DE 3229125**
**04.08.82 DE 3229126**

(43) Veröffentlichungstag der Anmeldung:
**07.03.84 Patentblatt 84/10**

(84) Benannte Vertragsstaaten:
**AT BE CH DE FR GB IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Fengler, Gerd, Dr.**
**Bethelstrasse 16**
**D-4150 Krefeld(DE)**

(72) Erfinder: **Arlt, Dieter, Prof. Dr.**
**Rybniker Strasse 2**
**D-5000 Köln 80(DE)**

(72) Erfinder: **Grohe, Klaus, Dr.**
**Am Wasserturm 10**
**D-5068 Odenthal(DE)**

(72) Erfinder: **Zeiler, Hans-Joachim, Dr.**
**Elsbeekerstrasse 15**
**D-5620 Velbert 15(DE)**

(72) Erfinder: **Metzger, Karl Georg**
**Pahlkestrasse 15**
**D-5600 Wuppertal 1(DE)**

(54) **4H-1,4-Benzothiazin-Derivate, Verfahren zu ihrer Herstellung und ihre Verwendung als Arzneimittel, Futterzusatzstoffe und Konservierungsmittel.**

(57) Die vorliegende Erfindung betrifft 4H-1,4-Benzothiazin-Derivate, ein Verfahren zu ihrer Herstellung, ihre Verwendung als Arzneimittel, insbesondere ihre Verwendung als Antiinfektiva, als Mittel zur Förderung des Wachstums und zur Verbesserung der Futterverwertung bei Tieren und als Konservierungsmittel. Es wurde gefunden, daß die neuen 4H-1,4-Benzothiazin-Derivate der allgemeinen Formel (I)

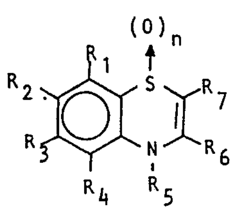

(I)

entsprechen.

EP 0 101 933 A1

BAYER AKTIENGESELLSCHAFT 5090 Leverkusen, Bayerwerk

Zentralbereich
Patente, Marken und Lizenzen Je-ABc

# 4H-1,4-Benzothiazin-Derivate, Verfahren zu ihrer Herstellung und ihre Verwendung als Arzneimittel, Futterzusatzstoffe und Konservierungsmittel

Die vorliegende Erfindung betrifft 4H-1,4-Benzothiazin-Derivate, ein Verfahren zu ihrer Herstellung, ihre Verwendung als Arzneimittel, insbesondere ihre Verwendung als Antiinfektiva, als Mittel zur Förderung des Wachstums und zur Verbesserung der Futterverwertung bei Tieren und als Konservierungsmittel. Es wurde gefunden, daß die neuen 4H-1,4-Benzothiazin-Derivate der allgemeinen Formel (I)

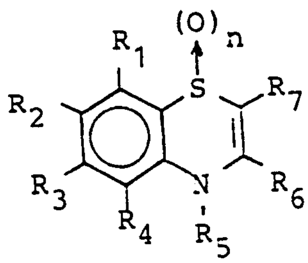

(I)

in welcher

n für 0, 1 oder 2 steht,

$R_1, R_4$ gleich oder verschieden sein können und Wasserstoff, gegebenenfallsusubstituiertes Alkyl oder Halogen bedeuten,

$R_2, R_3$ gleich oder verschieden sein können und für Wasserstoff, gegebenenfalls substituiertes Alkyl, gegebenenfalls substituiertes Aryl, gegebenenfalls substituiertes Heterocyclyl, Halogen, Cyano, Nitro, gegebenenfalls substituiertes Amino, Alkoxy oder Alkoxycarbonyl stehen.

$R_1$, $R_2$, $R_3$ und $R_4$ sollen nicht gleichzeitig Wasserstoff bedeuten.

$R_5$ und $R_6$ gleich oder verschieden sein können und Wasserstoff, gegebenenfalls substituiertes Alkyl oder gegebenenfalls substituiertes Cycloalkyl bedeuten und

$R_6$ noch für Alkoxycarbonyl und Carboxyl steht,

$R_7$ für Wasserstoff, gegebenenfalls substituiertes Alkyl, Cyano, Alkoxycarbonyl, Carboxyl, Alkyl- oder Arylcarbonyl steht,

wobei $R_6$ und $R_7$ auch einen gegebenenfalls substituierten 5- bis 6-gliedrigen Carbocyclus schließen können.

Verbindungen dieser allgemeinen Formel (I) weisen eine starke und breite antimikrobielle Wirksamkeit auf und

verbessern das Wachstum und die Futterverwertung bei Tieren, sie sind sehr breit als Antiinfektiva einsetzbar.

Ebenfalls Gegenstand der vorliegenden Erfindung sind 4H-1,4-Benzothiazin-Derivate der allgemeinen Formel (Ia)

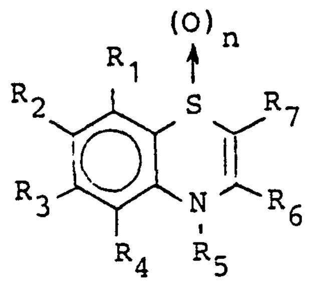

(Ia)

in welcher

$n, R_1, R_2, R_3, R_4, R_5, R_6$ und $R_7$ die in der allgemeinen Formel (I) angegebene Bedeutung besitzen, wobei $R_1, R_2, R_3$ und $R_4$ auch gleichzeitig Wasserstoff bedeuten können,

zur Verwendung bei der Bekämpfung von Krankheiten.

Weiterhin wurde gefunden, daß man die 4H-1,4-Benzothiazin-Derivate der allgemeinen Formeln (I) und (Ia) erhält, wenn man die offenkettigen Verbindungen der allgemeinen Formel (II)

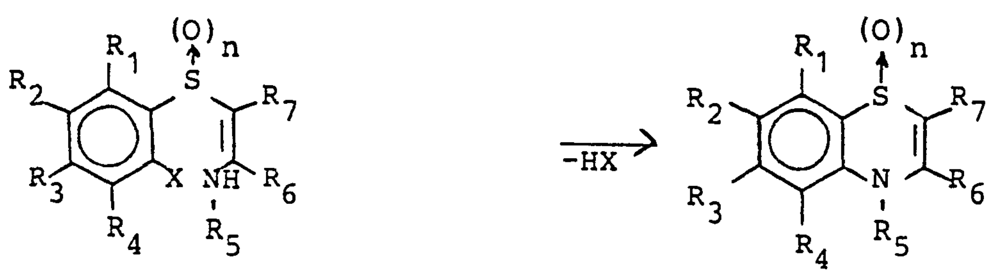


(II) (I)

in welcher

$n, R_1, R_2, R_3, R_4, R_5, R_6$ und $R_7$ die in der allgemeinen Formel (Ia) angegebene Bedeutung besitzen, wobei $R_6$ und/oder $R_7$ nicht Carboxyl bedeuten sollen und

X für Halogen, bevorzugt für Chlor oder Brom steht,

mit Basen in Gegenwart von Verdünnungsmitteln cyclisiert.

Verbindungen der allgemeinen Formel (Ia) mit $R_6$, $R_7$ = Carboxyl sind leicht aus den entsprechenden Alkoxycarbonylderivaten durch Verseifung gewinnbar.

In der allgemeinen Formel (Ia) steht gegebenenfalls substituiertes Alkyl $R_1$, $R_2$, $R_3$, $R_4$, $R_5$, $R_6$ und $R_7$ für geradkettiges oder verzweigtes Alkyl mit 1 bis 4 Kohlenstoffatomen. Beispielhaft seien gegebenenfalls substituiertes Methyl, Ethyl, n- und i-Propyl, n-, i- und t-Butyl genannt.

Gegebenenfalls substituiertes Cycloalkyl $R_5$, $R_6$ ist monocyclisch und enthält 3 bis 7, insbesondere 3 bis 6 C-Atome. Beispielhaft seien gegebenenfalls substituiertes Cyclopropyl, Cyclobutyl, Cyclopentyl und Cyclohexyl genannt.

Als gegebenenfalls substituiertes Aryl $R_2$, $R_3$ steht gegebenenfalls substituiertes Phenyl, Substituenten stehen in o-, m- und p-Stellung.

Als gegebenenfalls substituiertes Heterocyclyl $R_2$, $R_3$ stehen heteroparaffinische, heteroaromatische oder

heteroolefinische 5- bis 7-gliedrige, vorzugsweise 5- oder 6-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Als Beispiele seien gegebenenfalls substituiertes Thienyl, Furyl, Oxazolyl, Isoxazolyl, Thiazolyl, Isothiazolyl, Pyrrolyl, Imidazolyl, Pyrazolyl, Oxdiazolyl, Thiadiazolyl, Triazolyl, Oxtriazolyl, Thiatriazolyl, Tetrazolyl, Pyridyl, Pyrazinyl, Pyrimidinyl, Tetrahydrofuranyl, Dioxanyl, Pyrrolidinyl, Piperazinyl, Piperidinyl und Morpholinyl, genannt.

Als gegebenenfalls substituiertes Amino $R_2$, $R_3$ steht Mono- und Dialkylamino.

Alkoxy bedeutet bevorzugt Methoxy und Ethoxy.

Alkylcarbonyl $R_7$ steht für Methyl- und Ethylcarbonyl, Arylcarbonyl $R_7$ steht für gegebenenfalls substituiertes Phenylcarbonyl. Halogen bedeutet Fluor, Chlor, Brom und Jod, bevorzugt Fluor, Chlor und Brom.

Gegebenenfalls substituierte Alkyl-, Cycloalkyl-, Aryl- und Heterocyclyl-Reste können einen oder mehrere, vorzugsweise 1 bis 3, insbesondere 1 oder 2 gleiche oder verschiedene Reste $R_8$ tragen, wobei $R_8$ für geradkettiges oder verzweigtes Alkyl mit vorzugsweise 1 bis 6, insbesondere 1 bis 4 C-Atomen, z.B. Methyl,

Ethyl, n- und i-Propyl, n-, i- und t-Butyl und $CF_3$, $CCl_3$ sowie für Aryl, z.B. Phenyl, Niederalkyl-oxy-, vorzugsweise $CH_3O$-, $C_2H_5O$-, sowie für Aryloxy, z.B. Phenoxy, weiterhin für Niederalkyl-thio, z.B. $CH_3S$-, $C_2H_5S$-, für HCO-NH-, für Diniederalkylamino, z.B. Dimethylamino, Diethylamino, für Niederalkyl-O-CO-, z.B. $CH_3O$-CO- und $C_2H_5O$-CO-, für Halogen, bevorzugt Fluor, Chlor, Brom, -C≡N, COOH und für $-NH_2$ sowie $NO_2$ steht.

Die erfindungsgemäßen 4H-1,4-Benzothiazin-Derivate der allgemeinen Formeln (I) und (Ia) zeigen überraschenderweise eine starke und breite antibakterielle Wirksamkeit.

Sie wirken sehr stark als Antiinfektiva allgemein, besonders bei der Bekämpfung bakterieller Infektionen.

Die unter die allgemeinen Formeln (I) und (Ia) fallenden Verbindungen lassen sich nach dem vorstehend beschriebenen Verfahren leicht herstellen.

Einige Verbindungen, die unter die allgemeine Formel (I) fallen, lassen sich nach den folgenden Verfahren herstellen:

a) 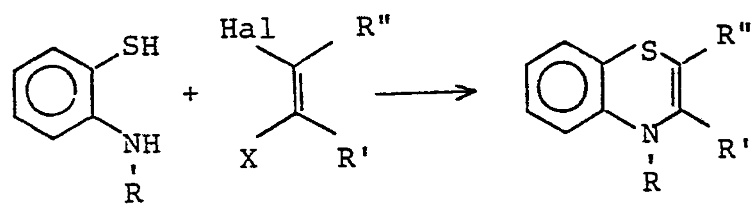

X = -OH, -NH Aryl, Halogen
Hal = Chlor, Brom,

R = Alkyl, R' = Wasserstoff, Alkoxycarbonyl, R'' = Wasserstoff, Alkyl, Alkoxycarbonyl

b) 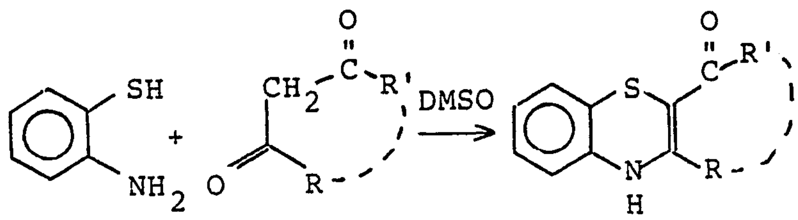


R = R' = Alkyl, R, R' schließen einen 6-gliedrigen Carbocyclus,

c) 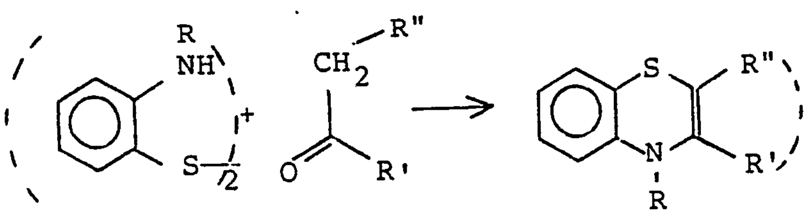

R = H, Alkyl, R' = R" = Alkyl, R', R" schließen einen 6-gliedrigen Carbocyclus

d) 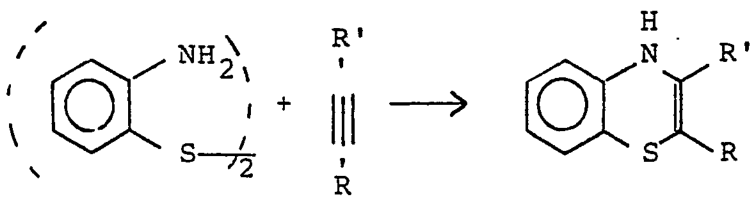

R = Alkylcarbonyl, Alkoxycarbonyl, R' = H, Phenyl, Alkoxycarbonyl,

e)

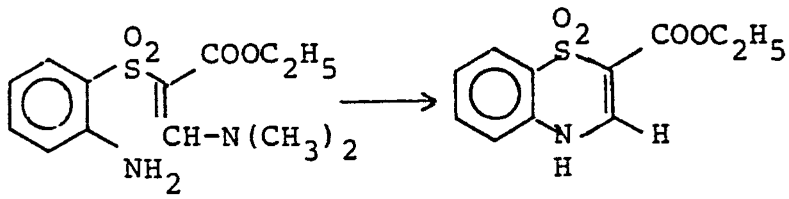

Die Methoden a) bis e) haben den Nachteil, daß sie die Synthese der gewünschten 4H-1,4-Benzothiazin-Derivate - gemäß der allgemeinen Formel (I) - in technisch befriedigender Weise nicht zulassen, weil sie den Einsatz speziell substituierter o-Amino-thiophenole (a), (b) bzw. der entsprechenden Disulfide (c), (d) oder von o-Nitrophenylsulfinaten (e) verlangen, die technisch nur sehr schwer zugänglich sind und deshalb einen erhöhten Aufwand darstellen. Darüber hinaus ist zur Herstellung N-alkylierter Verbindungen häufig noch ein Alkylierungsschritt nötig. Es bestand daher die Aufgabe, eine technisch befriedigende Synthese zu finden, die alle Substituentenmuster - gemäß der allgemeinen Formel (I) - einschließt.

Die Aufgabe wird gelöst durch die vorliegende Erfindung, die dadurch gekennzeichnet ist, daß man die erfindungsgemäßen 4H-1,4-Benzothiazin-Derivate der allgemeinen Formel (I) in einfacher Weise durch Cyclisierung der entsprechenden offenkettigen Verbindungen der allgemeinen Formel (V)

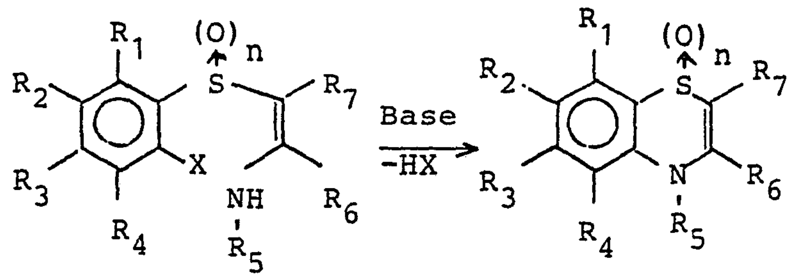


(V) (I)

in Gegenwart einer Base und von Verdünnungsmitteln erhält.

Verwendet man beispielsweise α-/(2-Chlor-5-nitrophenyl)-sulfenyl/-ß-ethylamino-acrylsäure-ethylester (E/Z-Gemisch) und Kalium-tert.-butylat als Ausgangsstoffe, so kann der Reaktionsablauf durch das folgende Formelschema wiedergegeben werden:

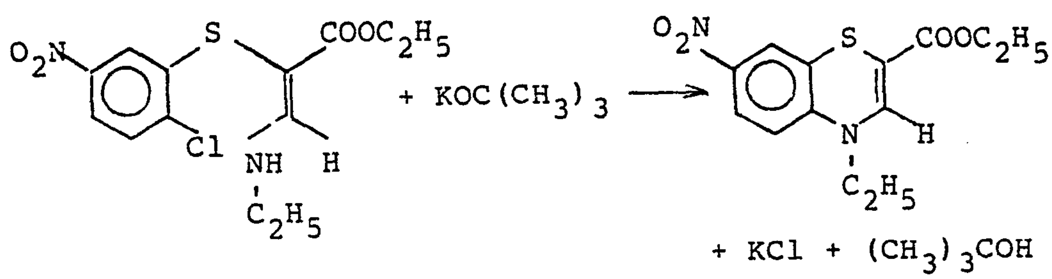


In den allgemeinen Formeln (I) und (V) stehen als gegegebenenfalls substituiertes Alkyl $R_1$, $R_2$, $R_3$, $R_4$, $R_5$, $R_6$ und $R_7$ geradkettige oder verzweigte Alkylreste mit 1 bis 6, vorzugsweise 1 bis 4 Kohlenstoffatomen. Beispielhaft seien gegebenenfalls substituiertes Methyl, Ethyl, n- und i-Propyl, n-, i- und t-Butyl genannt.

Gegebenenfalls substitutiertes Cycloalkyl $R_5$ und $R_6$ ist monocyclisch und enthält vorzugsweise 3 bis 7, besonders bevorzugt 3 bis 5 C-Atome. Beispielhaft seien gegebenenfalls substituiertes Cyclopropyl, Cyclobutyl und Cyclopentyl genannt.

Als gegebenenfalls substituiertes Aryl $R_2$ und $R_3$ steht gegebenenfalls substituiertes Phenyl. Substituenten im Phenylring stehen in o-, m- oder p-Stellung.

Als gegebenenfalls substituiertes Heterocyclyl $R_2$ und $R_3$ stehen heteroparaffinische, heteroaromatische oder heteroolefinische 5- bis 7-gliedrige, vorzugsweise 5- oder 6-gliedrige Ringe mit vorzugsweise 1 bis 3, besonders bevorzugt 1 oder 2 gleichen oder verschiedenen Heteroatomen. Als Heteroatome stehen Sauerstoff, Schwefel oder Stickstoff. Als Beispiele seien gegebenenfalls substituiertes Thienyl, Furyl, Oxazolyl, Isoxazolyl, Pyrazolyl, Oxdiazolyl, Thiadiazolyl, Triazolyl, Oxtriazolyl, Thiatriazolyl, Tetrazolyl, Pyridyl, Pyrazinyl, Pyrimidinyl, Tetrahydrofuranyl, Dioxanyl, Pyrrolidinyl, Piperazinyl, Piperidinyl und Morpholinyl, genannt.

Als gegebenenfalls substituiertes Amino $R_2$ und $R_3$ steht Monoalkylamino und Dialkylamino.

Alkoxy bedeutet vorzugsweise Methoxy oder Ethoxy.

Halogen steht für Fluor, Chlor, Brom und Jod, bevorzugt für Fluor, Chlor und Brom.

Alkylcarbonyl $R_7$ bedeutet geradkettiges oder verzweigtes Alkylcarbonyl mit vorzugsweise 1-4 C-Atomen, besonders

bevorzugt Methyl- und Ethylcarbonyl.

Arylcarbonyl $R_7$ steht für gegebenenfalls substituiertes Phenylcarbonyl.

Gegebenenfalls substituierte Alkyl-, Cycloalkyl-, Aryl- und Heterocyclyl-Reste können einen oder mehrere, vorzugsweise 1 bis 3, insbesondere 1 oder 2 gleiche oder verschiedene Reste $R_8$ tragen, wobei $R_8$ für geradkettiges oder verzweigtes Alkyl mit vorzugsweise 1 bis 6, besonders bevorzugt 1 bis 4 C-Atomen, z.B. Methyl, Ethyl, n- und i-Propyl, n-, i- und t-Butyl und $CF_3$, $CCl_3$ sowie für Aryl, z.B. Phenyl, Niederalkyl-oxy-, vorzugsweise $CH_3O$-, $C_2H_5O$-, sowie für Aryl-oxy, z.B. Phenoxy, weiterhin für Niederalkyl-thio, z.B. $CH_3S$-, $C_2H_5S$-, für HCO-NH-, für Diniederalkylamino, z.B. Dimethylamino, Diethylamino, für Niederalkyl-O-CO-, z.B. $CH_3O$-CO- und $C_2H_5O$-CO-, für Halogen, bevorzugt Fluor, Chlor, Brom, $-C{\equiv}N$, COOH und für $-NH_2$ sowie $NO_2$ steht.

In der allgemeinen Formel (V) steht X für Halogen, bevorzugt für Chlor und Brom.

Die erfindungsgemäß einzusetzenden Derivate der Formel (V), wobei n=O bedeutet, erhält man durch Umsetzung von Sulfensäurehalogeniden der allgemeinen Formel (VI) mit Enaminen der allgemeinen Formel (VII) in Gegenwart eines Säurefängers und von Verdünnungsmitteln.

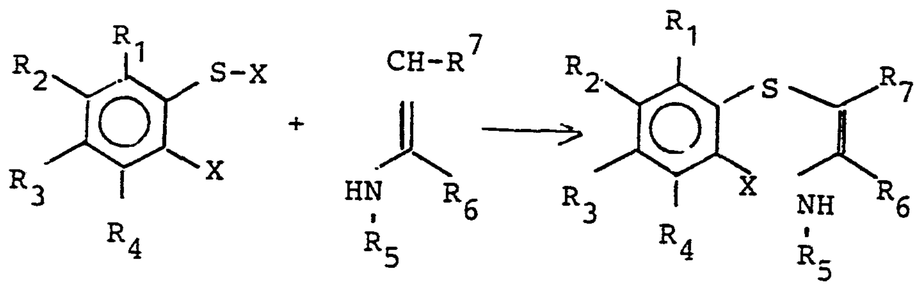

(VI) (VII) (V, n=O)

Die Verbindungen der allgemeinen Formeln (VI) und (VII), in der $R_1$, $R_2$, $R_3$, $R_4$, $R_5$, $R_6$ und $R_7$ die oben angegebene Bedeutung besitzen und X für Halogen steht, sind literaturbekannt oder lassen sich nach bekannten Verfahren herstellen (vergl. z.B.: E. Kühle, The Chemistry of the Sulfenic Acids, G. Thieme, Stuttgart 1973 und Organikum, VEB Deutscher Verlag der Wissenschaften, Berlin 1965, S. 371-372).

Zur Darstellung der Ausgangsmaterialien der Formel (V, n=O) legt man zweckmäßigerweise (VII) in einem Verdünnungsmittel vor, das gleichzeitig Säurefänger ist wie z.B. Pyridin oder Triethylamin und gibt (VI) in äquimolarer Menge hinzu. Die Reaktionstemperatur kann zwischen 0°C und 200°C, vorzugsweise zwischen 20°C und 100°C liegen, dabei arbeitet man im allgemeinen bei Normaldruck. Die Aufarbeitung der Reaktionsansätze zur Isolierung von (V, n=O) erfolgt in allgemein bekannter Art und Weise.

Die Derivate (V, n=O) fallen dabei als E/Z-Isomerengemische an, beide Formen sind aber als Startmaterial für das erfindungsgemäße Verfahren geeignet.

Die entsprechenden Sulfoxide und Sulfone der Formel (V, n=1 bzw. 2) erhält man aus (V, n=O) in allgemein bekannter Weise durch Oxidation mit geeigneten Oxidationsmitteln (vergl. z.B.: C. Ferri, Reaktionen der organischen Synthese, C. Thieme Verlag, Stuttgart 1978, S. 470).

Als erfindungsgemäß verwendbare Basen kommen beispielsweise metallorganische Reagenzien wie Lithiumdiisopropylamid, Butyl-lithium, Phenyl-lithium oder Grignard-Verbindungen wie Methylmagnesiumiodid oder Alkalialkoholate wie Kalium-tert.-butylat, Natriummethylat und -ethylat sowie Stickstoffbasen wie Diazabicycloundecan und Lutidin sowie Alkalicarbonate in Betracht.

Als Verdünnungsmittel kommen alle inerten organischen Lösungsmittel in Frage. Hierzu gehören vorzugsweise Ether wie Dioxan und Tetrahydrofuran sowie dipolar aprotische Solventien wie Dimethylsulfoxid, Dimethylformamid und N-Methylpyrrolidon. Das erfindungsgemäße Verfahren kann durchgeführt werden in Gegenwart von ausschließlich einem oder mehreren Lösungsmitteln.

Die Reaktionstemperaturen können in einem größeren Bereich variiert werden. Im allgemeinen arbeitet man zwischen 0°C und +250°C, vorzugsweise zwischen 20°C und 200°C.

Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens setzt man die Ausgangsstoffe in äquimolaren Mengen ein.

Die Aufarbeitung der Reaktionsansätze zur Isolierung der erfindungsgemäßen Verbindungen erfolgt in durchweg allgemein bekannter Art und Weise.

Die nach dem erfindungsgemäßen Verfahren herstellbaren 4H-1,4-Benzothiazin-Derivate der allgemeinen Formel (I) können als antibakterielle Mittel sowie als Lipoxygenasehemmer verwendet werden, außerdem stellen sie wertvolle Zwischenprodukte für Pharmaka und Pflanzenschutzmittel dar.

Verwendet man beispielsweise α-[(2-Chlor-5-nitro-phenyl)-sulfenyl]-ß-ethylamino-acrylsäuremethylester und Kalium-tert.-butylat als Ausgangsmaterialien, so kann der Reaktionsablauf durch das folgende Formelschema wiedergegeben werden:

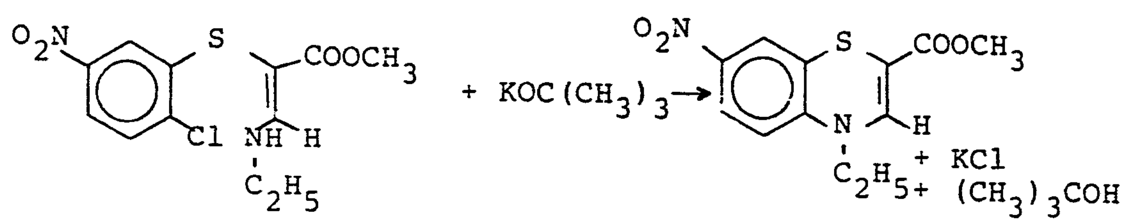

Die Verbindungen der allgemeinen Formel (II, n = O) in welcher

$R_1$, $R_2$, $R_3$, $R_4$, $R_5$, $R_6$ und $R_7$ die in (Ia) angegebene Bedeutung haben, aber $R_6$, $R_7$ = Carboxyl ausgeklammert sein

soll, und X für Halogen, bevorzugt für Chlor und Brom steht, sind durch Umsetzung von Sulfensäurehalogeniden der Formel (III) mit Enaminen der Formel (IV)

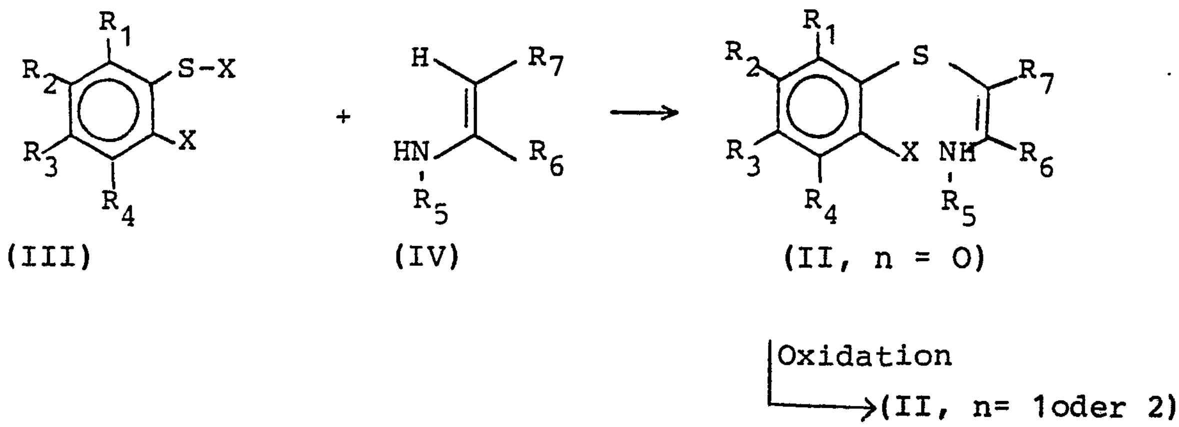


(III) (IV) (II, n = 0)

(II, n= 1oder 2)

worin

$R_1, R_2, R_3, R_4, R_5, R_6, R_7$ sowie X die oben angegebene Bedeutung haben,

in Gegenwart von Basen und gegebenenfalls Verdünnungsmitteln erhältlich. Die Verbindungen (II, n = 1 oder 2) sind aus (II, n = 0) durch Oxidation mit geeigneten Oxidationsmitteln in allgemein üblicher Weise gewinnbar (vgl. C. Ferri, Reaktionen der organischen Synthese, G. Thieme Verlag, Stuttgart 1978, S. 470).

Die Derivate (III) und (IV) sind teilweise bekannt oder lassen sich nach bekannten Verfahren herstellen (vgl. E. Kühle, The Chemistry of the Sulfenic Acids, G. Thieme Verlag, Stuttgart 1973 und Organikum, VEB Deutscher Verlag der Wissenschaften, Berlin 1965, S. 371-372).

Zur Darstellung von (II, n = O) legt man das Enamin der Formel (IV) zweckmäßig in einem Verdünnungsmittel vor, das gleichzeitig Base ist, beispielsweise Pyridin oder Triethylamin, und gibt das Sulfensäurehalogenid in äquimolarer Menge zu. Man arbeitet bei Normaldruck, die Reaktionstemperaturen können in einem größeren Bereich variiert werden. Man arbeitet im allgemeinen zwischen 0°C und +200°C, vorzugsweise zwischen 20°C und 100°C.

Die Aufarbeitung der Reaktionsansätze zur Isolierung der Ausgangsstoffe (II) für das erfindungsgemäße Verfahren erfolgt durchweg in allgemein bekannter Art und Weise.

Die Derivate (II) fallen dabei als E/Z-Isomerengemische an, beide Formen sind aber als Startmaterial für das erfindungsgemäße Verfahren geeignet.

Als erfindungsgemäß verwendbare Basen kommen beispielsweise metallorganische Reagenzien wie Lithiumdiisopropylamid, Butyl-lithium, Phenyl-lithium oder Grignard-Verbindungen wie Methylmagnesiumiodid oder Alkalialkoholate wie Kalium-tert.-butylat, Natriummethylat und -ethylat sowie Stickstoffbasen wie Diazabicycloundecan und Lutidin sowie Alkalicarbonate in Betracht.

Als Verdünnungsmittel kommen alle inerten organischen Lösungsmittel in Frage. Hierzu gehören vorzugsweise Ether wie Dioxan und Tetrahydrofuran sowie dipolar

aprotische Solventien wie Dimethylsulfoxid, Dimethylformamid und N-Methylpyrrolidon. Das erfindungsgemäße Verfahren kann in Gegenwart von ausschließlich einem oder mehreren Lösungsmitteln durchgeführt werden.

Die Reaktionstemperaturen können in einem größeren Bereich variiert werden. Im allgemeinen arbeitet man zwischen 0°C und +250°C, vorzugsweise zwischen 20°C und 200°C.

Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens setzt man die Ausgangsstoffe in äquimolaren Mengen ein.

Die Aufarbeitung der Reaktionsansätze zur Isolierung der erfindungsgemäßen Verbindungen erfolgt in allgemein bekannter Art und Weise.

Die erfindungsgemäßen Verbindungen zeigen ein breites antibakterielles Spektrum gegen gram-positive und gramnegative Keime, insbesondere gegen Enterobakteriaceae, die gegen andere Antibiotika oder Chemotherapeutika resistent sind.

Die erfindungsgemäßen Verbindungen weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, (Staph. = Staphylococcus); Lactobacteriaceae, wie Streptokokken, z.B. Streptococcus pyogenes, α-bzw. ß-hämolysierende Streptokokken, nicht

($\gamma$-)-hämolysierende Streptokokken, Enterokokken und Dipolococcus pneumoniae (Pneumokokken) (Str.=Streptococcus); Enterobacteriaceae, wie Escherichiae-Bakterien der Coli-Gruppe: Escherichia-Bakterien, z.B. Escherichia coli, Enterobacter-Bakterien, z.B. E.aerogenes, E. cloacae, Klebsiella-Bakterien, z.B. K.pneumoniae, Serratia, z.B. Serratia marcescens (E. = Enterobacter) (K. = Klebsiella), Proteae-Bakterien der Proteus-Gruppe: Proteus, z.B. Proteus vulgaris, Pr.morganii, Pr.rettgeri, Pr.mirabilis, (Pr.= Proteus);

Pseudomonadaceae, wie Pseudomonas-Bakterien, z.B. Pseudomonas aeruginosa, (PS.=Pseudomonas);

Bacteroidaceae, wie Bacteroides-Bakterien, z.B. Bacteroides fragilis, (B. = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, sie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylakohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylakohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeührten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/ oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 5 bis etwa 1000, vorzugsweise 10 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gram-negative oder grampositive Bakterien verhindert, gebessert und/oder geheilt werden und ebenso eine Förderung des Wachstums und eine Verbesserung der Verwertung des Futters erreicht werden.

Die neuen Verbindungen zeichnen sich durch starke antibakterielle Wirkungen, die in vivo und in vitro geprüft wurden, und durch orale Resorbierbarkeit aus.

Die erfindungsgemäßen Verbindungen können zum Zwecke der Erweiterung des Wirkungsspektrums und um eine Wirkungssteigerung zu erzielen mit anderen antimikrobiellen Wirkstoffen z.B. mit Penicillinen, Cephalosporinen oder anderen Betalactamen, kombiniert werden.

Die erfindungsgemäßen Verbindungen können zum Zweck der Erweiterung des Wirkungsspektrums und um eine Wirkungssteigerung zu erreichen auch mit anderen Antibiotika z.B. Aminoglykosiden, wie z.B. Gentamicin, Sisomicin, Kanamicin, Amikacin oder Tobramicin, Tetracyclinen oder Novobiocin kombiniert werden.

Die Wirksamkeit der erfindungsgemäßen Präparate kann durch die folgenden in vitro-Versuche beispielhaft demonstriert werden:

In vitro-Versuche

Die Verbindungen der Beispiele 19, 20 und 21 wurden im Agarverdünnungstest auf antibakterielle Wirkung geprüft. Die Konzentration betrug 128 und 8 mcg pro Milliliter Agar.

Bei folgenden Bakterienstämmen wurde völlige Wachstumshemmung festgestellt (MHK; mcg/ml)

| Beispiel Nr. | Staph 133 | Streptococ. W | Klebsiella 8085 |
|---|---|---|---|
| 19 | | ≤128<br>>8 | |
| 20 | 128<br>>8 | ≤128<br>>8 | ≤128<br>>8 |
| 21 | | ≤128<br>>8 | |

Die vorliegende Erfindung betrifft weiterhin neue, substituierte Enamine, ein Verfahren zu ihrer Herstellung sowie ihre Verwendung als Zwischenprodukte für die Synthese pharmakologisch einsetzbarer 4H-1,4-Benzothiazin-Derivate.

Die neuen, substituierten Enamine entsprechen der allgemeinen Formel (VIII)

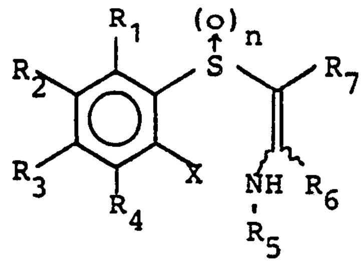

(VIII)

in welcher

n für 0, 1 oder 2 steht,

$R_1$, $R_4$ gleich oder verschieden, H, gegebenenfalls substituiertes Alkyl oder Halogen bedeuten,

$R_2$,$R_3$ gleich oder verschieden, für H, gegebenenfalls substituiertes Alkyl, gegebenenfalls substituiertes Aryl, gegebenenfalls substituiertes Heterocyclyl, Halogen, gegebenenfalls substituiertes Amino, Nitro, Cyano, Alkoxycarbonyl oder Alkoxy stehen,

$R_5$,$R_6$ gleich oder verschieden, H, gegebenenfalls substituiertes Alkyl oder gegebenenfalls substituiertes Cycloalkyl bedeuten, und

$R_7$ für H, gegebenenfalls substituiertes Alkyl, Cyano, Alkoxycarbonyl, Alkyl- oder Arylcarbonyl steht, wobei $R_6$ und $R_7$ auch einen 5- bis 6-gliedrigen, gegebenenfalls substituierten Carbocyclus schließen können und

X Halogen bedeutet.

Besonders bevorzugt sind substituierte Enamine der Formel (VIIIa)

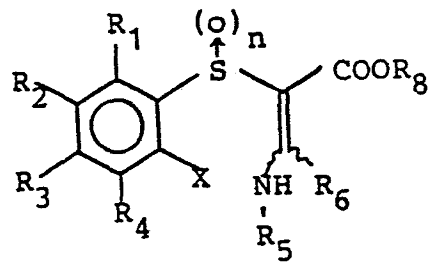

(VIIIa)

in welcher

n, $R_1$, $R_2$, $R_3$, $R_4$, $R_5$, $R_6$ und X die in Formel (I) angegebene Bedeutung haben und

$R_8$ für gegebenenfalls substituiertes Alkyl steht.

Die erfindungsgemäßen Verbindungen der allgemeinen Formel (VIII) können als E- oder Z-Isomer vorliegen, deshalb sollen beide Formen sowie das Gemisch zum Gegenstand der vorliegenden Erfindung gehören.

In den allgemeinen Formeln (VIII) und (VIIIa) stehen als gegebenenfalls substituiertes Alkyl $R_1$, $R_2$, $R_3$, $R_4$, $R_5$, $R_6$, $R_7$ und $R_8$ geradkettige oder verzweigte Alkylreste mit bis zu 4 Kohlenstoffatomen. Beispielhaft seien gegebenenfalls substituiertes Methyl, Ethyl, n- und i-Propyl, n-, i- und t-Butyl genannt.

Gegebenenfalls substitutiertes Cycloalkyl $R_5$ und $R_6$ ist monocyclisch und enthält vorzugsweise 3 bis 7, insbesondere 3 bis 5 C-Atome. Beispielhaft seien gegebenenfalls substituiertes Cyclopropyl, Cyclobutyl und Cyclopentyl genannt.

Als gegebenenfalls substituiertes Aryl $R_2$ und $R_3$ steht gegebenenfalls substituiertes Phenyl. Substituenten im Phenylring stehen in o-, m- oder p-Stellung.

Als gegebenenfalls substituiertes Heterocyclyl $R_2$ und $R_3$ stehen heteroparaffinische, heteroaromatische oder heteroolefinische 5- bis 7-gliedrige, vorzugsweise 5- oder 6-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Als Beispiele seien gegebenenfalls substituiertes Thienyl, Furyl, Oxazolyl, Isoxazolyl, Pyrazolyl, Oxdiazolyl, Thiadiazolyl, Triazolyl, Oxtriazolyl, Thiatriazolyl, Tetrazolyl, Pyridyl, Pyrazinyl, Pyrimidinyl, Tetrahydrofuranyl, Dioxanyl, Pyrrolidinyl, Piperazinyl, Piperidinyl und Morpholinyl, genannt.

Als gegebenenfalls substituiertes Amino $R_2$ und $R_3$ steht Monoalkylamino und Dialkylamino.

Alkoxy bedeutet Methoxy oder Ethoxy.

Halogen steht für Fluor, Chlor, Brom und Jod, bevorzugt für Fluor, Chlor und Brom.

Alkylcarbonyl $R_7$ bedeutet geradkettiges oder verzweigtes Alkylcarbonyl mit vorzugsweise 1 - 4 C-Atomen, insbesondere Methyl- und Ethylcarbonyl.

Arylcarbonyl $R_7$ steht für gegebenenfalls substituiertes Phenylcarbonyl.

Gegebenenfalls substituierte Alkyl-, Cycloalkyl-, Aryl- und Heterocyclyl-Reste können einen oder mehrere, vorzugsweise 1 bis 3, insbesondere 1 oder 2 gleiche oder verschiedene Reste $R_8$ tragen, wobei $R_8$ für geradkettiges oder verzweigtes Alkyl mit vorzugsweise 1 bis 6, insbesondere 1 bis 4 C-Atomen, z.B. Methyl, Ethyl, n- und i-Propyl, n-, i- und t-Butyl und $CF_3$, $CCl_3$ sowie für Aryl, z.B. Phenyl, Niederalkyl-oxy-, vorzugsweise $CH_3O$-, $C_2H_5O$-, sowie für Aryl-oxy, z.B. Phenoxy, weiterhin für Niederalkyl-thio, z.B. $CH_3S$-, $C_2H_5S$-, für HCO-NH-, für Diniederalkylamino, z.B. Dimethylamino, Diethylamino, für Niederalkyl-O-CO-, z.B. $CH_3O$-CO- und $C_2H_5O$-CO-, für Halogen, bevorzugt Fluor, Chlor, Brom, -C N, COOH und für $-NH_2$ sowie $NO_2$ steht.

Die erfindungsgemäßen Verbindungen der allgemeinen Formel (VIII), für n = O, lassen sich durch Umsetzung von Sulfensäurehalogeniden der Formel (IX) mit Substanzen der Formel (X)

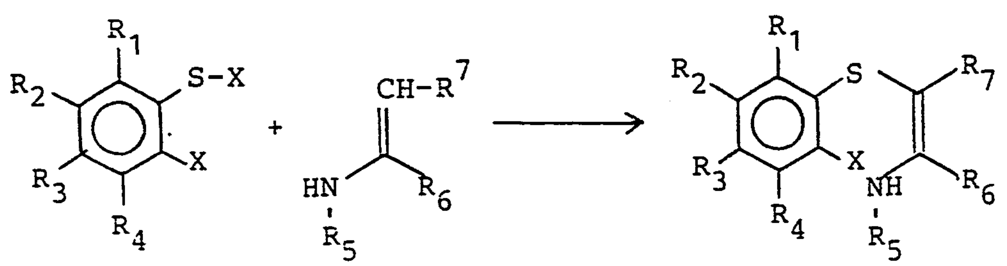

(IX) (X) (VIII) für n = O

worin
$R_1$, $R_2$, $R_3$, $R_4$, $R_5$, $R_6$, $R_7$ und X die in der allgemeinen Formel (VIII) angegebene Bedeutung haben,

in Gegenwart von Basen und gegebenenfalls Verdünnungsmitteln herstellen.

Die Verbindungen der allgemeinen Formeln (IX) und (X) sind literaturbekannt oder lassen sich nach bekannten Verfahren herstellen (vgl. z.B.: E.Kühle, The Chemistry of the Sulfenic Acids, G. Thieme, Stuttgart 1973 und Organikum, VEB Deutscher Verlag der Wissenschaften, Berlin 1965, S. 371 - 372).

Verwendet man beispielsweise 2,4-Dichlor-phenylsulfenylchlorid und ß-Ethylamino-acrylsäure-methylester als Ausgangsstoffe, so kann der Reaktionsablauf durch das folgende Formelschema wiedergegeben werden:

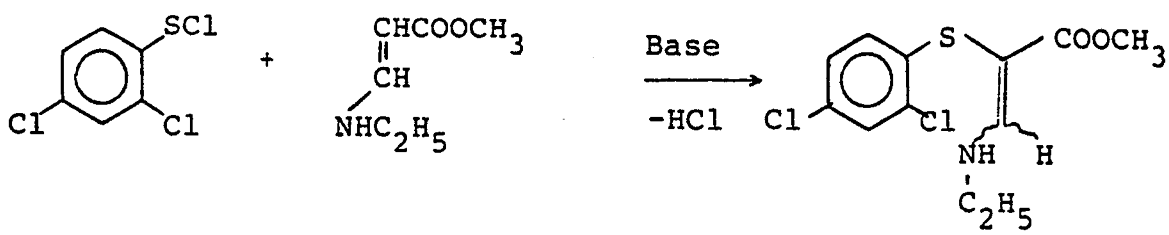


Als Basen kommen anorganische und organische Basen in Frage.

Als Beispiele für anorganische Basen seien Natrium-, Kalium- und Calciumhydroxid, Alkalicarbonate und Alka-

lihydrogencarbonate genannt. Als organische Basen kommen beispielhaft tertiäre Amine, vorzugsweise Niederalkylamine, z.B. Triethylamin, und/oder cyclische Basen, z.B. Pyridin in Frage.

Als Verdünnungsmittel kommen alle inerten organischen Lösungsmittel in Frage. Hierzu gehören vorzugsweise Kohlenwasserstoffe, wie Benzol und Toluol, chlorierte Kohlenwasserstoffe, wie Methylenchlorid, Chloroform, Tetrachlorkohlenstoff, 1,2-Dichlorethan, 1,2,2-Trichlorethan und Chlorbenzol, sowie Ether wie Diethylether, Dioxan oder Tetrahydrofuran. Das erfindungsgemäße Verfahren kann durchgeführt werden in Gegenwart von ausschließlich einem oder mehreren organischen Lösungsmitteln oder Wasser oder einem oder mehreren mit Wasser nicht mischbaren Lösungsmitteln.

Bevorzugt sind Verdünnungsmittel, die gleichzeitig Basen darstellen, beispielsweise Pyridin oder Triethylamin.

Die Reaktionstemperaturen können in einem größeren Bereich variiert werden. Im allgemeinen arbeitet man zwischen etwa 0°C und etwa 200°C, vorzugsweise zwischen 20°C und 100°C.

Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens

werden die Reaktionspartner bevorzugt in äquimolaren Mengen zur Reaktion gebracht.

Die Aufarbeitung der Reaktionsansätze zur Isolierung der erfindungsgemäßen Verbindungen erfolgt bekannter Art und Weise.

Die erfindungsgemäßen Verbindungen der allgemeinen Formel (VIII), für n = 1 oder 2, erhält man aus den Substanzen (VIII), für n = 0, durch Oxidation mit geeigneten Oxidationsmitteln.

Verwendet man beispielsweise α-/(2,4-Dichlorphenyl)-sulfenyl/-ß-ethylamino-acrylsäuremethylester und m-Chlorperbenzoesäure als Ausgangsstoffe, so kann der Reaktionsablauf durch das folgende Formelschema beschrieben werden:

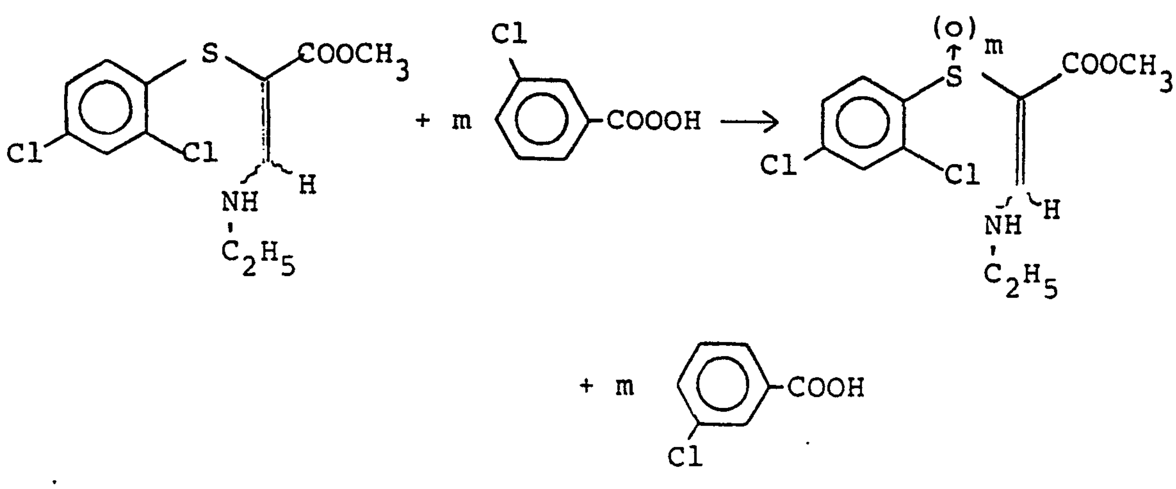

m = 1, 2

Die erfindungsgemäß verwendbaren Oxidationsmittel sind ebenfalls bekannt, beispielhaft seien genannt Wasserstoffperoxid, Perameisensäure, Peressigsäure, Perbenzoesäure, m-Chlorperbenzoesäure, Natrium-metaperjodat sowie Luftsauerstoff. Weitere geeignete Oxidationsmittel sind in C.Ferri, Reaktionen der organischen Synthese (C.Thieme Verlag, Stuttgart 1978, S. 470) beschrieben.

Als Verdünnungsmittel kommen alle inerten organischen Lösungsmittel in Frage. Hierzu gehören vorzugsweise chlorierte Kohlenwasserstoffe, wie Methylenchlorid, Chloroform, Tetrachlorkohlenstoff, 1,2-Dichlorethan, 1,2,2-Trichlorethan und Chlorbenzol, Alkohole, vorzugsweise Methanol, Ethanol sowie Isopropanol, niedere Fettsäuren, bevorzugt Ameisensäure, Essigsäure und Propionsäure und Wasser. Das erfindungsgemäße Verfahren kann durchgeführt werden in Gegenwart von ausschließlich einem oder mehreren Lösungsmitteln.

Die Reaktionstemperaturen können in einem größeren Bereich variiert werden. Im allgemeinen arbeitet man zwischen etwa -20°C und etwa +100°C, vorzugsweise zwischen 0°C und +60°C. Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens setzt man zur Darstellung der Sulfoxide ((I), n=1), bzw.

der Sulfone ((VIII), n=2) die jeweils angemessene Menge an Oxidationsmittel ein.

Die Aufarbeitung der Reaktionsansätze zur Isolierung der erfindungsgemäßen Verbindungen erfolgt durchweg in allgemein bekannter Art und Weise.

Die erfindungsgemäßen Derivate der Formel (VIII) sind wichtige Zwischenprodukte für die Synthese pharmakologisch einsetzbarer 4H-1,4-Benzothiazin-Derivate.

Beispiel 1

Herstellung von α-[(Pentachlorphenyl)-sulfenyl]-ß-ethylamino-acrylsäuremethylester

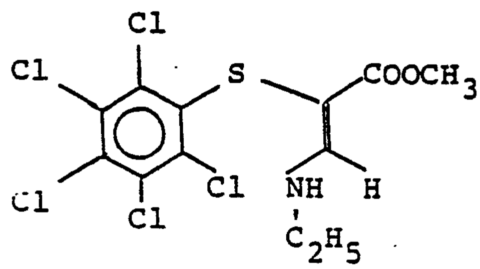

0.1 Mol ß-Ethylamino-acrylsäuremethylester werden in 50 ml absolutem Pyridin vorgelegt und 0.1 Mol Pentachlorphenylsulfenylchlorid portionsweise zugegeben. Danach wird noch 15 Min. auf 60°C erhitzt und anschließend das Pyridin im Vakuum abgezogen. Der Rückstand wird in Dichlormethan und Wasser aufgenommen; die organische Phase wird abgetrennt, mehrfach mit Wasser gewaschen und dann eingeengt. Der Rückstand wird aus Aceton umkristallisiert.

Fp.: 135°C
Ausbeute: 53 % der Theorie.

Analog wurden hergestellt:

| Beispiel Nr. | Formel | Fp. [°C] | Ausbeute [% d. Th.] |
|---|---|---|---|
| 2 | 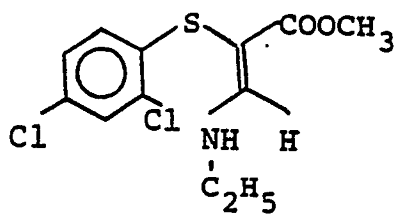 | 97-101 | 48 |
| 3 | 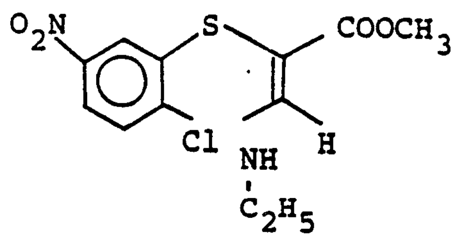 | 94-97 | 86 |
| 4 | 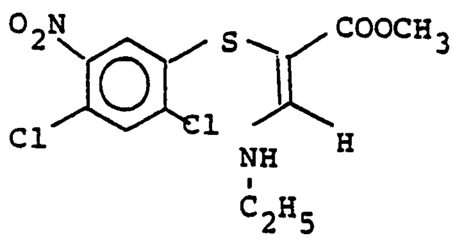 | 186-189 | 66 |
| 5 | 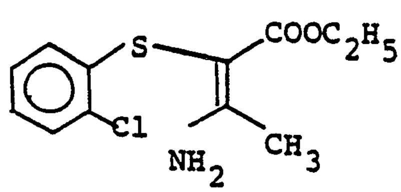 | 83-84 | 68 |

Beispiel 6

Darstellung von α-[(2,4-Dichlor-5-nitro-phenyl)-sulfinyl]-ß-ethylamino-acrylsäuremethylester

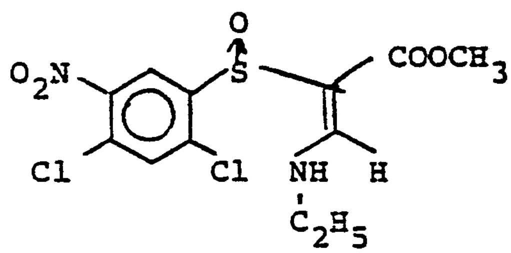

Man legt 0.01 Mol α-[(2,4-Dichlor-5-nitro-phenyl)-sulfenyl]-ß-ethylamino-acrylsäuremethylester in 100 ml Dichloromethan vor und gibt bei Raumtemperatur 0.01 Mol (bzw. 0.02 Mol für das Sulfon) m-Chlorperbenzoesäure portionsweise zu. Nach 48 Stunden bei Raumtemperatur wird mit Bicarbonatlösung neutralisiert, die organische Phase abgetrennt und eingeengt. Der Rückstand wird mit Ether kristallisiert.

Fp.: 140-143°C
Ausbeute: 92 % der Theorie.

Analog wurden dargestellt:

| Beispiel Nr. | Formel | Fp. [°C] | Ausbeute [% d. Th.] |
|---|---|---|---|
| 7 | 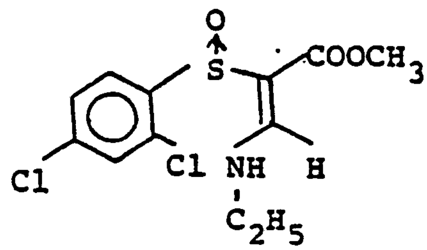 | 120-123 | 43 |
| 8 | 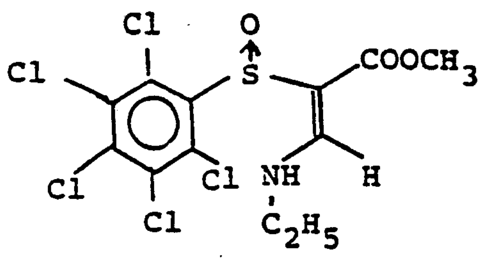 | 172-174 | 35 |
| 9 | 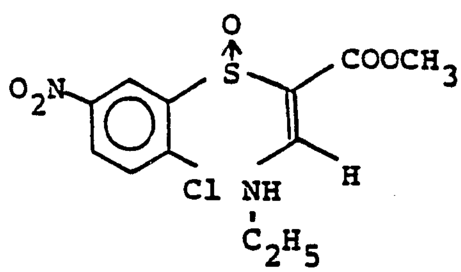 | 193-195 | 76 |
| 10 | 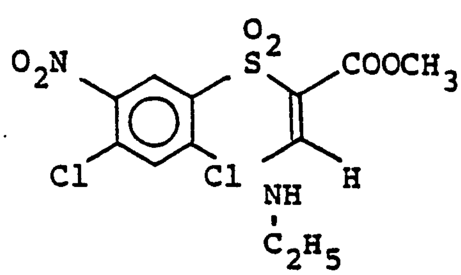 | 150-155 | 69 |

Beispiel 11

Darstellung von 2-Methoxycarbonyl-4-ethyl-5,6,7,8-tetrachlor-4H-1,4-benzothiazin-1-oxid

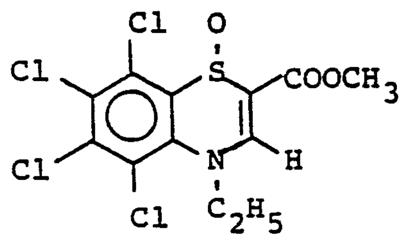

Man legt 2.13 g (0.005 Mol) α-[(Pentachlorphenyl)-sulfinyl]-ß-ethylamino-acrylsäuremethylester in 50 ml absolutem Dioxan vor und gibt bei Raumtemperatur 2.3 ml 20 %ige Butyl-lithiumlösung in Hexan zu. Nach 10 Stunden bei Raumtemperatur wird das Dioxan abgezogen, der Rückstand in Wasser/Dichlormethan aufgenommen und die organische Phase abgetrennt. Nach Abziehen des Solvens hinterbleibt ein viskoses Öl, das mit Ether kristallisiert werden kann.

Fp.: 157-158°C,
Ausbeute: 1.23 g (≙ 63 % der Theorie).

Beispiel 12

Darstellung von 2-Methoxycarbonyl-4-ethyl-7-nitro-4H-1,4-Benzothiazin

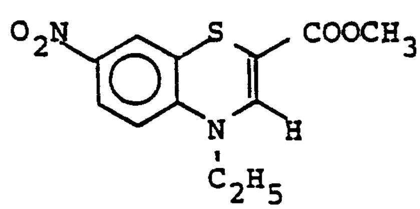

Man legt 0.01 Mol α-[(5-Nitro-phenyl)-sulfenyl]-β-ethylamino-acrylsäuremethylester in 50 ml absolutem Dimethylsulfoxid vor und gibt bei Raumtemperatur 0.01 Mol frisch sublimiertes Kalium-tert.-butylat portionsweise zu. Nach 8 Stunden bei 100°C wird das Dimethylsulfoxid abgezogen, der Rückstand in Wasser/Dichloromethan aufgenommen und die organische Phase abgetrennt. Nach Einengen wird der Rückstand mit Ether kristallisiert.

Fp.: 162-166°C,
Ausbeute: 90 % der Theorie.

Analog wurden dargestellt:

| Beispiel Nr. | Formel | Fp. [°C] | Ausbeute [% d. Th.] |
|---|---|---|---|
| 13 | 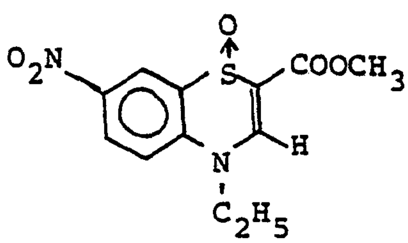 | 185-189 | 85 |
| 14 | 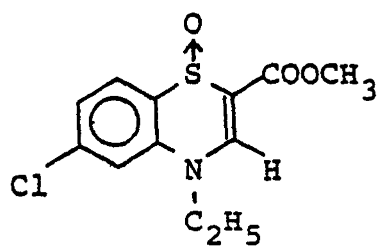 | 172-175 | 90 |

| Beispiel Nr. | Formel | Fp. /°C/ | Ausbeute /% d. Th./ |
|---|---|---|---|
| 15 | 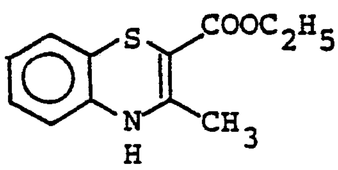 | 145 | 15 |
| 16 | 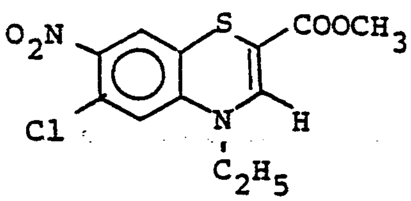 | 163-166 | 76 |
| 17 | 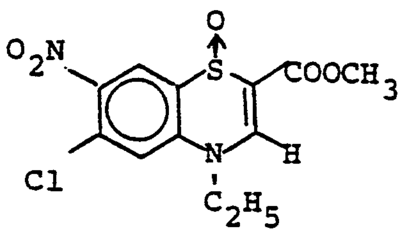 | 157-162 | 85 |
| 18 | 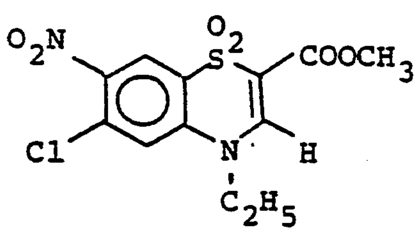 | 178-180 | 47 |

| Beispiel Nr. | Formel | Fp. (°C) | Ausbeute (% d.Th.) |
|---|---|---|---|
| 19 | 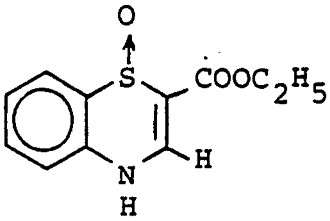 | 188-192 | 70 |
| 20 | 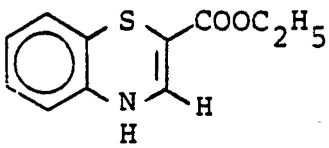 | 140-142 | 38 |

Beispiel 21

Darstellung von 4-Ethyl-5,6,7,8-tetrachlor-4H-1,4-benzothiazin-1-oxid-2-carbonsäure

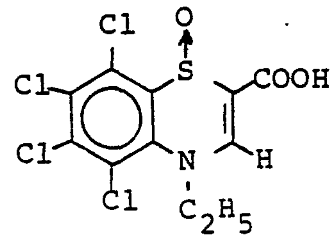

Man löst 1,95 g (0,005 Mol) des entsprechenden Methylesters in einem Gemisch aus 10 ml Dioxan und 10 ml 6 %iger Natronlauge auf und erwärmt 4 Stunden auf 50°C. Anschließend werden Wasser und Dioxan abgezogen, der Rückstand in Wasser aufgenommen und die Lösung mit konzentrierter Salzsäure angesäuert. Der Niederschlag kann mit Ether kristallisiert werden.
Fp.: 226-228°C.
Ausbeute: 0,74 g (≙ 40 % der Theorie)
analog gewinnt man:

Beispiel 22

4-Ethyl-7-nitro-4H-1,4-benzothiazin-2-carbonsäure

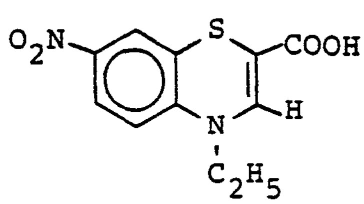

Fp.: 193-195°C
Ausbeute: 56 % der Theorie.

Patentansprüche

1. 4H-1,4-Benzothiazin-Derivate der allgemeinen Formel (I)

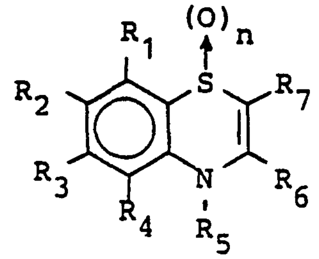

(I)

in welcher

n für 0, 1 oder 2 steht,

$R_1$,$R_4$ gleich oder verschieden sein können und jeweils für Wasserstoff, gegebenenfalls substituiertes Alkyl oder Halogen stehen

$R_2$,$R_3$ gleich oder verschieden sein können und jeweils für Wasserstoff, gegebenenfalls substituiertes Alkyl, gegebenenfalls substituiertes Aryl, gegebenenfalls substituiertes Heterocyclyl, Halogen, Cyano, Nitro, gegebenenfalls substituiertes Amino, Alkoxy oder Alkoxycarbonyl stehen

$R_5$,$R_6$ gleich oder verschieden sein können, und jeweils für Wasserstoff, gegebenenfalls substituiertes Alkyl oder gegebenenfalls substituiertes Cycloalkyl bedeuten und

$R_6$ für Alkoxycarbonyl oder Carboxyl steht,

$R_7$ für Wasserstoff, gegebenenfalls substituiertes Alkyl, Cyano, Alkoxycarbonyl, Carboxyl, Alkylcarbonyl oder Arylcarbonyl steht,

$R_6$ und $R_7$ können auch einen gegebenenfalls substituierten 5- bis 6-gliedrigen Carbocyclus schließen

jedoch mit der Einschränkung, daß $R_1$, $R_2$, $R_3$ und $R_4$ nicht gleichzeitig Wasserstoff bedeuten.

2. 4H-1,4-Benzothiazin-Derivate der allgemeinen Formel (I) gemäß Anspruch 1 in welcher $R_1$ und $R_4$ gleichzeitig Wasserstoff bedeuten.

3. 4H-1,4-Benzothiazin-Derivate der allgemeinen Formel (Ia)

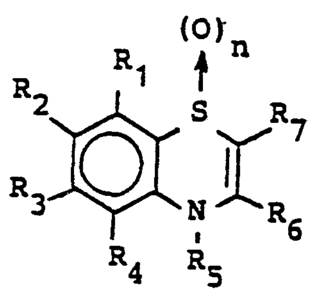

(Ia)

in welcher

n für 0, 1 oder 2 steht,

$R_1$,$R_4$ gleich oder verschieden sein können und jeweils für Wasserstoff, gegebenenfalls substituiertes Alkyl oder Halogen stehen

$R_2$,$R_3$ gleich oder verschieden sein können und jeweils für Wasserstoff, gegebenenfalls substituiertes Alkyl, gegebenenfalls substituiertes Aryl, gegebenenfalls substituiertes Heterocyclyl, Halogen, Cyano, Nitro, gegebenenfalls substituiertes Amino, Alkoxy oder Alkoxycarbonyl stehen

$R_5$,$R_6$ gleich oder verschieden sein können, und jeweils für Wasserstoff, gegebenenfalls substituiertes Alkyl oder gegebenenfalls substituiertes Cycloalkyl bedeuten und

$R_6$ für Alkoxycarbonyl oder Carboxyl steht,

$R_7$ für Wasserstoff, gegebenenfalls substituiertes Alkyl, Cyano, Alkoxycarbonyl, Carboxyl, Alkylcarbonyl oder Arylcarbonyl steht,

$R_6$ und $R_7$ können auch einen gegebenenfalls substituierten 5- bis 6-gliedrigen Carbocyclus schließen

zur Verwendung bei der Bekämpfung von Krankheiten.

4. Verbindungen der allgemeinen Formel (Ia) gemäß Anspruch 3 zur Verwendung als Antiinfektiva.

5. Arzneimittel enthaltend mindestens eine Verbindung der allgemeinen Formel (Ia) gemäß Anspruch 3.

6. Verfahren zur Herstellung von Verbindungen der allgemeinen Formel (Ia) gemäß Anspruch 3, dadurch gekennzeichnet, daß man Verbindungen der allgemeinen Formel (II)

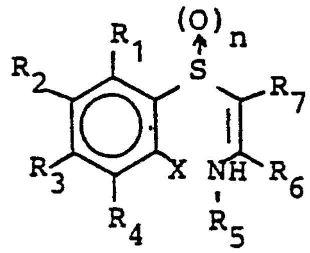

(II)

wobei

| | |
|---|---|
| n, $R_1$, $R_2$, $R_3$, $R_4$, $R_5$, $R_6$ und $R_7$ | die in Anspruch 3 angegebene Bedeutung besitzen, und |
| X | für Halogen, bevorzugt Chlor und Brom steht, |

mit Basen in Gegenwart von Verdünnungsmittel cyclisiert.

7. Verfahren zur Herstellung von 4H-1,4-Benzothiazin-Derivaten der allgemeinen Formel I

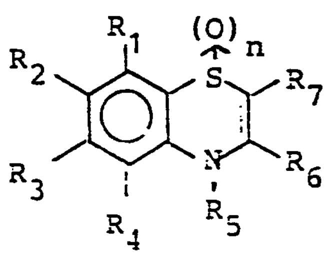

. (I)

in welcher

n für 0, 1 oder 2 steht,

$R_1$, $R_4$ gleich oder verschieden sein können und Wasserstoff, gegebenenfalls substituiertes Alkyl oder Halogen bedeuten,

$R_2$, $R_3$ gleich oder verschieden sein können und für Wasserstoff, gegebenenfalls substituiertes Alkyl, gegebenenfalls substituiertes Aryl, gegebenenfalls substituiertes Heterocyclyl, Halogen, gegebenenfalls substituiertes Amino, Nitro, Cyano, Alkoxycarbonyl oder Alkoxy stehen,

$R_5$, $R_6$ gleich oder verschieden sein können und Wasserstoff, gegebenenfalls substituiertes Alkyl, oder gegebenenfalls substituiertes Cycloalkyl bedeuten, und

$R_7$ für Wasserstoff, gegebenenfalls substituiertes Alkyl, Cyano, Alkoxycarbonyl, Alkyl- oder Arylcarbonyl steht, wobei $R_6$ und $R_7$ auch einen 5- bis 6-gliedrigen, gegebenenfalls substituierten Carbocyclus schließen können,

dadurch gekennzeichnet, daß man offenkettige Derivate der Formel (V)

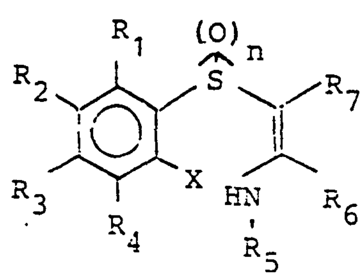

(V)

in welcher
n, $R_1$, $R_2$, $R_3$, $R_4$, $R_5$, $R_6$ und $R_7$ die oben angegebene Bedeutung haben und X Halogen bedeutet,

mit Basen in Gegenwart inerter Verdünnungsmittel bei Temperaturen zwischen 0°C und 250°C umsetzt.

8. Substituierte Enamine der allgemeinen Formel (VIII)

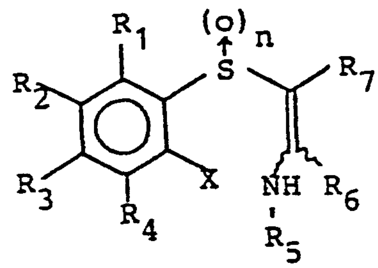

(VIII)

in welcher

n für 0, 1 oder 2 steht,

$R_1$, $R_4$ gleich oder verschieden sein können und Wasserstoff, gegebenenfalls substituiertes Alkyl oder Halogen bedeuten,

$R_2$, $R_3$ gleich oder verschieden sein können und für Wasserstoff, gegebenenfalls substituiertes Alkyl, gegebenenfalls substituiertes Aryl, gegebenenfalls substituiertes Heterocyclyl, Halogen, gegebenenfalls substituiertes Amino, Nitro, Cyano, Alkoxycarbonyl oder Alkoxy stehen,

$R_5$, $R_6$ gleich oder verschieden sein können und Wasserstoff, gegebenenfalls substituiertes Alkyl oder gegebenenfalls substituiertes Cycloalkyl bedeuten,

$R_7$ für Wasserstoff, gegebenenfalls substituiertes Alkyl, Cyano, Alkoxycarbonyl, Alkylcarbonyl, oder Arylcarbonyl steht, wobei $R_6$ und $R_7$ auch einen 5- bis 6-gliedrigen, gegebenenfalls substituierten Carbocyclus schließen können, und

X Halogen bedeutet.

9. Substituierte Enamine nach Anspruch 8 , gekennzeichnet durch die allgemeine Formel (VIIIa)

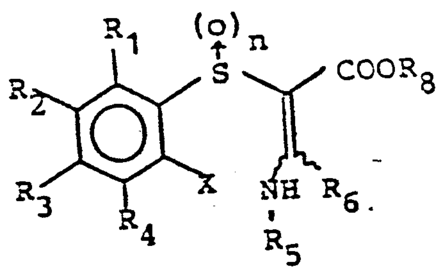

(VIIIa)

in welcher

n, $R_1$, $R_2$, $R_3$, $R_4$, $R_5$, $R_6$ und X die in Anspruch 8 angegebene Bedeutung haben und $R_8$ gegebenenfalls substituiertes Alkyl bedeutet.

10. Verfahren zur Herstellung substituierter Enamine der allgemeinen Formel (VIII), für n = 0, dadurch gekennzeichnet, daß man Sulfensäurehalogenide der allgemeinen Formel (IX)

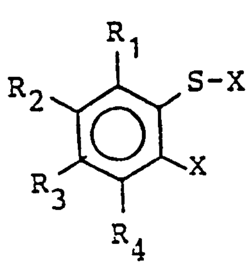

(IX)

in welcher

$R_1$, $R_2$, $R_3$, $R_4$ und X die in Anspruch 8 angegebene Bedeutung haben,

mit Verbindungen der allgemeinen Formel (X)

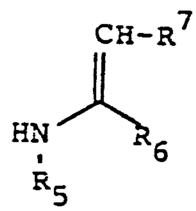

(X)

in welcher

$R_5$, $R_6$ und $R_7$ die in Anspruch 8 angegebene Bedeutung haben,

in Gegenwart von Basen und gegebenenfalls Verdünnungsmitteln umsetzt.

0101933

Europäisches Patentamt

# EUROPÄISCHER RECHERCHENBERICHT

Nummer der Anmeldung: EP 83107354.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP - A1 - 0 055 917 (FUJISAWA PHARMACEUTICAL CO. LTD)<br>* Anspruch 1 *<br>-- | 1,5 | C 07 D 279/16<br>C 07 C 149/42<br>C 07 C 149/43<br>A 61 K 31/54//<br>A 23 B 4/14<br>A 23 K 1/00 |
| A,P | EP - A1 - 0 061 082 (BAYER AG)<br>* Ansprüche 1,2 *<br>-- | 1,6 | |
| A,P | EP - A1 - 0 061 639 (BAYER AG)<br>* Anspruch 1; Seite 2, Zeilen 23-26 *<br>-- | 1 | |
| A | BE - A - 653 101 (LABORATOIRES S.M.B.)<br>* Anspruch 1 *<br>---- | 8,9 | |

RECHERCHIERTE SACHGEBIETE (Int. Cl. 3)

C 07 D 279/00
C 07 C 149/00

Der vorliegende Recherchenbericht wurde für alle Patentansprüche erstellt.

| Recherchenort | Abschlußdatum der Recherche | Prüfer |
|---|---|---|
| WIEN | 28-10-1983 | BRUS |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument